# EUROPEAN PATENT APPLICATION

(11) **EP 4 756 017 A1**
(43) Date of publication of application: **10.06.2026**
(21) Application number: 24218283.0
(22) Date of filing: 09.12.2024
(51) Int. Cl.: C12N 9/10, C12P 13/10, C12N 15/63, C12N 15/70, C12N 15/77, C12N 15/78, C12P 13/04

(54) **GUANIDINOACETIC ACID WITH REDUCED AMOUNTS OF OR NO TOXIC COMPONENTS**

(71) Applicant: Evonik Operations GmbH, 45128 Essen (DE)
(72) Inventor: MERZ, Juliane, 44139 Dortmund (DE); JANKOWITSCH, Frank, 48336 Sassenberg (DE); DENZ, Tobias, 60594 Frankfurt am Main (DE); NORDSCHILD, Anja, 61440 Oberursel (DE); REHREN, Claus, 63743 Aschaffenburg (DE); RICHERT, Susann, 63776 Mömbris (DE); BAUER, Lukas, 63874 Dammbach (DE); MARIN, Kay, 33829 Borgholzhausen (DE)
(74) Representative: Evonik Patent Association

(57) **Abstract**

The present invention relates to guanidinoacetic acid, characterized in that it comprises less than 0.3 ppm of melamine and/or less than 600 ppm of dicyandiamide and/or cyanamide as impurity or secondary component, determined by LC-MS/MS, a process for its preparation, a feed additive comprising or consisting of the guanidinoacetic acid according to the present invention or of the guanidinoacetic acid obtained by the process according to the present invention.

## Description

Guanidinoacetic acid (GAA) is a colorless, organic compound used as feed additive for animals. GAA is a direct natural precursor of creatine, which is active in vertebrate energy homeostasis. GAA is used in feed formulations of broiler and swine to improve feed conversion rates, animal health and meat quality. Therefore, the supplementation of GAA allows for an optimal supply of creatine in the organism, which has a positive influence on the energy transport in the muscle cells.

The first production of GAA was described in 1861 by addition of cyanamide to glycine. A weakly alkaline aqueous solution of ammonia was used as the reaction medium. Alternatively, a solution of sodium hydroxide or sodium carbonate was also used as base for pH adjustment. As until today, GAA is produced according to this old approach starting from the fossil carbon sources cyanamide and glycine. One drawback of producing GAA according to this old procedure is the formation of the side products melamine and dicyanamide which both have negative impact on living organisms. Dicyanamide and melamine are produced as side products by dimerization or trimerization of cyanamide (Thomas Güthner; Bernd Mertschenk (2006), Ullmann's Encyclopedia of Industrial Chemistry, Weinheim, Wiley-VCH). Dicyandiamide and melamine are toxic to humans and therefore highly regulated (see for example Scientific Opinion on Melamine in Food and Feed, EFSA Panel on Contaminants in the Food Chain (CONTAM) and EFSA Panel on Food Contact Materials, Enzymes, Flavourings and Processing Aids (CEF), first published: 13 April 2010, https://doi.org/10.2903/j.efsa.2010.1573). The International Agency for Research on Cancer (IARC) classified melamine as a possible carcinogen in 2017 (Yann Grosse, Dana Loomis, Kathryn Z Guyton, Fatiha El Ghissassi, Véronique Bouvard, Lamia Benbrahim-Tallaa, Heidi Mattock, Kurt Straif: Some chemicals that cause tumours of the urinary tract in rodents. In: The Lancet Oncology. 18, 2017, S. 1003-1004, doi:10.1016/S1470-2045(17)30505-3). At the beginning of July 2010, the World Health Organization set a maximum limit of 2.5 milligrams of melamine in one kilogram of food and pet food in its Codex Alimentarius. For infant food such as milk powder, a limit of 1 milligram of melamine per kilogram has been determined. In the European Union, the maximum levels of melamine in food are regulated by Regulation (EC) No. 1881/2006. The maximum limits are also based on what can be achieved through good manufacturing practice or good agricultural practice. In infant formula and follow-on formulae, the limit is 1 mg/kg, in all other foods it is 2.5 mg/kg. However, melamine is often present in traces in different food and feed types and thus, the risk is given, that the recommended daily dose is exceeded.

Consequently, it would be a clear improvement in the quality of GAA and the product itself, if the GAA did not have the potential to cause cancer due to traces of melamine and/or dicyandiamide.

It was found that these problems are solved by the GAA according to the present invention.

One object of the present invention is therefore guanidinoacetic acid, characterized in that it comprises less than 0.3 ppm of melamine and/or less than 600 ppm of dicyandiamide and/or cyanamide as impurity or secondary component, determined by LC-MS/MS.

In the context of the present invention the term secondary component is used to denote any further and thus secondary component in addition to the first and therefore primary component guanidinoacetic acid.

In the guanidinoacetic acid production, the by-product dicyandiamide is typically formed as by-product in significantly higher quantities than melamine. Nevertheless, melamine is the critical component, since its presence is regulated on a ppm scale, depending on the country. In principle, it is possible to purify the final GAA product in a wash step, but the high effort involved also results in high product and raw materials losses, which are lost with the wash water. The present invention also solves this problem, in that the guanidinoacetic acid according to the present invention comprises less than 0.3 ppm of melamine, preferably even not more than 0.1 ppm of melamine.

In an embodiment the guanidinoacetic acid according to the present invention comprises not more than 0.1 ppm of melamine, determined by LC-MS/MS.

Cyanamide is an organic compound with the formula CN₂H₂ and widely used in agriculture. Under the trade name dormex^{®}, it is a common agricultural rest-breaking agent applied in spring to stimulate uniform opening of buds, early foliation, and bloom. Cyanamide can effectively compensate for the moderate lack of chilling units accumulated in the previous autumn and save the harvest that would otherwise be lost.

Dicyandiamide, also known as 2-cyanoguanidine is an organic compound C₂N₄H₄ from the family of cyanamides. Chemically, it belongs to the cyanamide and guanidine derivatives. It is a nitrile derived from guanidine, and it is also a dimer of cyanamide, from which it can be prepared as well.

Melamine is an organic compound with the formula C₃H₆N₆. It is a trimer of cyanamide, with a 1,3,5-triazine skeleton. Melamine is susceptible to hydrolysis, which yields other triazine derivatives, such as ammeline or ammelide.

Ammeline (4,6-diamino-2-hydroxy-1,3,5-triazine) is a triazine derivative. It is the first step on melamine hydrolysis. Further hydrolysis yields ammelide (6-amino-2,4-dihydroxy-1 ,3,5-triazine).

Since the guanidinoacetic acid according to the present invention comprises less than 0.3 ppm of melamine, preferably even not more than 0.1 ppm of melamine, the guanidinoacetic acid according to the present invention also comprises very low levels of ammeline and/or ammelide.

In one embodiment the guanidinoacetic acid according to the present invention comprises less than 0.1 ppm of ammeline, determined by LC-MS/MS.

In another embodiment the guanidinoacetic acid according to the present invention comprises less than 0.1 ppm of ammelide, determined by LC-MS/MS.

Preferably, the guanidinoacetic acid according to the present invention comprises less than 0.1 ppm of ammeline and/or ammelide, determined by LC-MS/MS.

In yet another embodiment the guanidinoacetic acid according to the present invention comprises less than 0.1 ppm of cyanuric acid, determined by LC-MS/MS.

In a further embodiment the guanidinoacetic acid according to the present invention comprises less than 500 ppm of dicyandiamide and/or cyanamide, determined by LC-MS/MS.

In principle, the guanidinoacetic acid according to the present invention is not limited to a specific process for its preparation, provided that thus prepared guanidinoacetic acid comprises less than 0.3 ppm of melamine and/or less than 600 ppm of dicyandiamide and/or cyanamide as impurity or secondary component, determined by LC-MS/MS. It was found that a biosynthesis route is particularly suitable for preparing the guanidinoacetic acid with the features according to the present invention. The major biosynthetic step to form GAA is catalyzed by L-arginine:glycine amidinotransferase (AGAT), which transfers an amidino group from L-arginine to glycine to form GAA and L-ornithine. This enzyme can be used in a heterologous host to construct a basic GAA production strain. The L-ornithine can be readily recycled to L-arginine in the metabolism of bacteria. Therefore, the guanidinoacetic acid according to the present invention is in principle not subject to any limitations regarding the process for its production, provided that said process involves the cultivation of a microorganism comprising at least one gene coding for a protein having the function of an L-arginine:glycine amidinotransferase and that at least one carbon source comprising biogenic carbon is present.

Another object of the present invention is therefore a process for producing guanidinoacetic acid according to the present invention, comprising the step of cultivating a microorganism comprising at least one gene coding for a protein having the function of an L-arginine:glycine amidinotransferase in the presence of one or more carbon sources, wherein at least one carbon source comprises biogenic carbon.

In principle, the bacterial cell can form the precursor, L-arginine and glycine from glucose and ammonia. Thus, GAA can be formed directly from glucose and ammonia. Another option is that one or both substrates are fed into the fermentation medium. Both substrates, L-arginine and glycine can be taken up into the bacteria cell via transmembrane transport proteins and used by AGAT to form GAA.

In one embodiment of the process according to the present invention the at least one carbon source comprising biogenic carbon comprises or consists of a sugar, preferably a monosaccharide, in particular glucose.

The final content of biogenic carbon in the guanidinoacetic acid depends on the raw material, i.e., the at least one carbon source used in the process for its production. Renewable carbon sources come from plants and thus, provide the guanidinoacetic acid with the highest possible amount of biogenic carbon. Hence, the higher is the overall amount of the renewable carbon source in the process for GAA preparation, the higher is the content of biogenic carbon in the produced GAA. Notwithstanding, it is possible in the context of the present invention that the GAA is produced from carbon sources, which differ in their biogenic carbon content, provided that at least one carbon source comprises biogenic carbon.

In the context of the present invention, it is also possible to produce GAA using different carbon sources. The carbon sources can differ in their chemical structure, for example the carbon source can be glucose and/or glycine. The carbon sources can also differ in their content of biogenic carbon. Hence, they can be renewable carbon sources and non-renewable sources, provided that at least one carbon source comprises biogenic carbon. For example, the GAA can be prepared from glycine as the first carbon source and glucose as the second carbon source. Glycine provides 2 of the 3 carbon atoms present in the GAA and glucose provides the remaining 1 carbon atom present in the GAA. In a further example the glucose is from a renewable source and thus the at least one carbon source comprising biogenic carbon, while glycine is from a fossil source, and thus does not contain biogenic carbon. In this case, one should expect that the thus produced GAA comprises approximately one third of biogenic carbon. However, the GAA thus produced comprises 38% of biogenic carbon and thus, even 5% more than expected. This can be explained by endogenous glycine that is produced by the cell in addition to the endogenously fed glycine. Accordingly, even when the GAA is produced not only from carbon sources comprising only biogenic carbon, the thus produced GAA still contains up to 40% of biogenic carbon.

The process according to the present invention is not limited regarding a specific nitrogen source. Rather, any conceivable nitrogen source can be in principle used in the process according to the present invention. Suitable nitrogen sources are for example ammonia, and urea. In order to keep the content of non-biogenic carbon as low as possible, it is preferred to use ammonia as nitrogen source in the process according to the present invention.

In another embodiment of the process according to the present invention, the cultivation is performed in the presence of a sugar, preferably a monosaccharide, in particular glucose, and ammonia.

One should expect that if only glucose (from a renewable source) and ammonia were used as feedstock in the process for producing the GAA, the thus obtained GAA would comprise 100% of biogenic carbon. However, in that case it was observed that the GAA only comprises up to 80% of biogenic carbon. It is believed that carbon isotopes can be fractionated differently towards metabolic side products. Very likely, the missing 20% of the biogenic carbon was shifted disproportionately towards the CO₂-offgas stream or the biomass side-product.

In one embodiment of the process according to the present invention the microorganism further comprises at least one gene coding for a protein having the function of an NADH-dependent amino acid dehydrogenase.

The activity of the NADH-dependent amino acid dehydrogenase is preferably increased compared with the respective activity in the wildtype microorganism. Preferably, the NADH-dependent amino acid dehydrogenase is selected from the group consisting of alanine dehydrogenase (EC 1.4.1.1), glycine dehydrogenase (EP 1.4.1.10), and aspartate dehydrogenase (EC 1.4.1.21). The protein having the function of an NADH-dependent amino acid dehydrogenase is preferably a heterologous protein.

It is preferred that the microorganism has an increased ability to produce L-arginine from L-ornithine compared to the respective enzymic activity in the wildtype microorganism.

Preferably, the microorganism further comprises an enzyme having the function of an ornithine carbamoyltransferase with an increased activity compared with the respective enzymic activity in the wildtype microorganism. The microorganism preferably further comprises an enzyme having the function of an argininosuccinate synthetase with an increased activity compared with the respective enzymic activity in the wildtype microorganism. The increased activities of the enzymes are preferably achieved by overexpressing the gene encoding the respective enzymes. Preferably, the activity of a protein having the function of a malate synthase is decreased compared with the respective activity in the wildtype microorganism, wherein the expression of a gene encoding the protein having the function of a malate synthase is preferably attenuated compared with the expression of the respective gene in the wildtype microorganism or a gene encoding the protein having the function of a malate synthase is preferably deleted. The expression of an *argR* gene coding for the arginine responsive repressor protein *ArgR* is preferably attenuated compared with the expression of the *argR* gene in the wildtype microorganism or an *argR* gene is preferably deleted. It is preferred that at least one or more of the genes coding for an enzyme of the biosynthesis pathway of L-arginine, comprising *gdh, argJ, argB, argC,* and/or *argD* coding for a glutamate dehydrogenase, an ornithine acetyltransferase, an acetylglutamate kinase, an acetylglutamylphosphate reductase, and for an acetylornithine aminotransferase, respectively is overexpressed.

Preferably, the gene coding for a protein having the function of an L-arginine:glycine amidinotransferase is heterologous.

The microorganism described in the paragraphs above preferably belongs to the genus *Corynebacterium,* to the genus *Enterobacteriaceae,* or to the genus *Pseudomonas.*

In another embodiment of the process according to the present invention the microorganism further comprises at least one protein having the function of a glyoxylate aminotransferase.

The enzymic activity of the at least one protein having the function of a glyoxylate aminotransferase is preferably increased compared with the enzymic activity in the wildtype microorganism. Preferably, the microorganism has an increased ability to produce L-arginine compared with the ability of the wildtype microorganism. In particular, the microorganism has increased activity of an enzyme having the function of a carbamoylphosphate synthase compared with the respective enzymic activity in the wildtype microorganism. The microorganism preferably further comprises an enzyme having the function of an argininosuccinate lyase with an increased activity compared with the respective enzymic activity in the wildtype microorganism. The microorganism preferably further comprises an enzyme having the function of an ornithine carbamoyltransferase with an increased activity compared with the respective enzymic activity in the wildtype microorganism. The microorganism preferably further comprises an enzyme having the function of an argininosuccinate synthetase with an increased activity compared with the respective enzymic activity in the wildtype microorganism. Preferably, the increased activities of the enzymes are achieved by overexpressing the genes encoding the respective enzymes. It is preferred that the activity of a protein having the function of a malate synthase is decreased compared to the respective activity in the wildtype microorganism, wherein the expression of a gene encoding the protein having the function of a malate synthase is preferably attenuated compared to the expression of the respective gene in the wildtype microorganism or wherein a gene encoding the protein having the function of a malate synthase is preferably deleted.

The arginine operon (*argCJBDFR*) is preferably overexpressed. It is preferred that the expression of an *argR* gene coding for the arginine responsive repressor protein *ArgR* is attenuated compared with the expression of the *argR* gene in the wildtype microorganism or the *argR* gene is deleted. Preferably, at least one of the genes coding for an enzyme of the biosynthetic pathway of L-arginine, comprising *gdh, argJ, argB, argC,* and/or *argD* coding for a glutamate dehydrogenase, an ornithine acetyltransferase, an acetylglutamate kinase, an acetylglutamylphosphate reductase, and for an acetylornithine aminotransferase, respectively, is overexpressed.

Preferably, the gene coding for a protein having the function of an L-arginine:glycine amidinotransferase is hetereologous. The gene coding for a protein having the function of an L-arginine:glycine amidinotransferase is preferably overexpressed.

The microorganism described in the paragraphs above preferably belongs to the genus *Corynebacterium,* to the genus *Enterobacteriaceae,* or to the genus *Pseudomonas.* It is further preferred that the microorganism described in the paragraphs above is *Corynebacterium glutamicum, Escherichia coli,* or *Pseudomonas putida.*

In a further embodiment of the process the activity of a protein having the function of a malate synthase is decreased compared with the respective activity in the wildtype microorganism.

It is preferred that the expression of gene encoding the protein having the function of a malate synthase is attenuated compared with the expression of the respective gene in the wildtype microorganism or a gene encoding the protein having the function of a malate synthase is deleted. The microorganism preferably further comprises an increased activity of an enzyme having the function of a glyoxylate aminotransferase compared with the respective activity in the wildtype microorganism. Preferably, the microorganism comprises at least one gene encoding a protein having the enzymic activity of a glyoxylate aminotransferase is overexpressed.

It is preferred that the microorganism has an increased ability to produce L-arginine compared with the ability of the wildtype microorganism, wherein the microorganism has increased activity of an enzyme having the function of a carbamoylphosphate synthase compared with the respective enzymic activity in the wildtype microorganism. Preferably, the microorganism further comprises an enzyme having the function of an argininosuccinate lyase with an increased activity compared with the respective activity in the wildtype microorganism. The microorganism preferably further comprises an enzyme having the function of an ornithine carbamoyltransferase with an increased activity compared with the respective enzymic activity in the wildtype microorganism. Preferably, the microorganism further comprises an enzyme having the function of an argininosuccinate synthetase with an increased activity compared with the respective enzymic activity in the wildtype microorganism. The increased activities of the enzymes are preferably achieved by overexpressing the genes encoding the respective enzymes.

It is further preferred that the arginine operon (*argCJBDFR*) is overexpressed. It is preferred that the expression of an *argR* gene coding for the arginine responsive repressor protein *ArgR* is attenuated compared with the expression of the *argR* gene in the wildtype microorganism or the *argR* gene is deleted. Preferably, the at least one of the gene coding for an enzyme of the biosynthetic pathway of L-arginine, comprising *gdh, argJ, argB, argC,* and/or *argD* coding for a glutamate dehydrogenase, an ornithine acetyltransferase, an acetylglutamate kinase, an acetylglutamylphosphate reductase, and for an acetylornithine aminotransferase, respectively, is overexpressed.

Preferably, the gene coding for a protein having the function of an L-arginine:glycine amidinotransferase is heterologous. The gene coding for a protein having the function of an L-arginine:glycine amidinotransferase is preferably overexpressed.

The microorganism described in the paragraphs above preferably belongs to the genus *Corynebacterium,* to the genus *Enterobacteriaceae,* or to the genus *Pseudomonas.* It is further preferred that the microorganism described in the paragraphs above is *Corynebacterium glutamicum, Escherichia coli,* or *Pseudomonas putida.*

A further object of the present invention is guanidinoacetic acid comprising less than 0.3 ppm of melamine and/or less than 600 ppm of dicyandiamide and/or cyanamide as impurity or secondary component, determined by LC-MS/MS, wherein said guanidinoacetic acid is obtained by the process according to the present invention.

The guanidinoacetic acid according to the present invention, and the guanidinoacetic acid obtained by the process according to the present invention can be incorporated into a feed additive and can be used as such or in the form of the feed additive for supplementing an animal diet.

A further object of the present invention is therefore a feed additive comprising or consisting of the guanidinoacetic acid according to the present invention or the guanidinoacetic acid obtained by the process according to the present invention.

Yet another object of the present invention is also a method for supplementing an animal feed, comprising the step of administering the guanidinoacetic acid according to the present invention, the guanidinoacetic acid obtained by the process according to the present invention, and/or the feed additive according to the present invention to an animal.

### Examples:

### I. Procedure for determination of dicyandiamide and melamine in guanidinoacetic acid

The determination of dicyandiamide and melamine in guanidinoacetic acid was done using liquid chromatography coupled with mass spectrometry (LC-MS). The identification of dicyandiamide and melamine is based on their retention time and the mass spectrometric experiment: the specific measurement in MRM mode (multi reaction monitoring). Due to electrospray ionization dicyandiamide was ionized to cation [M+H]⁺ at m/z = 85.1. This ion was isolated as precursor ion. After fragmentation in the applied triple quadrupole mass spectrometer the product was detected at m/z = 68.0 and monitored as selected ion chromatogram. Melamine was ionized to cation [M+H]⁺ at m/z = 127.1. This ion was isolated as precursor. After fragmentation, the product was detected at m/z = 85.1 and monitored as selected ion chromatogram.

### 1. Equipment

- LC-MS consisted of a triple stage quadrupole mass spectrometer "QQQ 6430" (Agilent Technologies) or equivalent coupled with an UPLC system "Infinity 1290 II" (Agilent Technologies) or equivalent with autosampler, degasser, and column heater. An UV detector is part of the HPLC, but not needed for these analyses.
- Analytical balance Mettler Toledo AX 205, or equivalent (minimum weight < 25 mg)
- Eppendorf Multipette E3x (equipped with 0.1 ml, 0.5 ml or 2.5 ml tips), or equivalent
- Vortexer "IKA VORTEX Genius 3", or equivalent

### 2. Chemicals and materials

- Guanidinoacetic acid (samples)
- Dicyandiamide 99%, D76609 (Sigma-Aldrich)
- Melamine 99%, M2659 (Sigma-Aldrich)
- Water, deionized with high purity (Sigma-Aldrich)
- Ammonium formiate, MS-grade (Sigma-Aldrich)
- Acetonitrile for chromatography, Lichrosolv (Merck)
- Trifluoroacetic acid, for analysis (Merck) 98 - 100%
- Chromatographic column: 50 x 2.1 mm Acclaim Trinity P2 (Thermo Fisher)

### 3. Operation procedure

### 3.1 Preparation of samples and reference solutions

| | |
|---|---|
| Diluent: | 20 mM ammoniumformiate in water / acetonitrile (5/95, v/v) |
| Solvent (2% TFA): | 25 trifluoroacetic acid in water |

### Calibration standards:

| | |
|---|---|
| Dicyandiamide: | For a stock solution 20 mg dicyandiamide were weighed in exactly into a 50 mL volumetric flask and filled to volume with water (SØ dicyandiamide). |
| Melamine: | For a stock solution 50 mg melamine were weight in exactly into a 10 mL volumetric flask and filled to volume with diluent (SØ melamine). |
| Dilution twice: | S1: 125 µL SØ dicyandiamide and/or 20 µL SØ melamine were transferred into a 10 mL volumetric flask and filled to volume with diluent. Concentration of each standard was about 100 µg/mL. |
| | S2: 50 µL S1 filled up to 10 mL; concentration was about 500 ng/mL |
| | S3: 1 mL S2 filled up to 10 mL; concentration was about 50 ng/mL |

The final calibration working solutions were prepared starting with the stock solutions S2 and/or S3. For this purpose, the pipetting scheme of table 1 was used:

**Table 1: Pipetting scheme for calibration steps.**

| | **Concentration level [ng/mL] (each standard)** | **S2 [µL]** | **S3 [µL]** | **Diluent [µL]** |
|---|---|---|---|---|
| Cal1 | 10 | | 200 | 800 |
| Cal2 | 25 | 50 | | 950 |
| Cal3 | 50 | 100 | | 900 |
| Cal4 | 100 | 200 | | 800 |
| Cal5 | 150 | 300 | | 700 |
| Cal6 | 200 | 400 | | 600 |
| Cal7 | 250 | 500 | | 500 |
| Cal8 | 300 | 600 | | 400 |

### Sample solutions:

| | |
|---|---|
| GAA solution: | for a stock solution 25 mg guanidinoacetic acid were weighed in exactly into a 10 mL volume flask and 5 mL solvent (2% TFA) were added. The sample was completely dissolved in an ultrasonic bath and then the flask was filled to volume with acetonitrile. (SØ guanidinoacetic acid; concentration: about 2.5 mg/mL). |
| Un-spiked samples*: | 20 µL SØ guanidinoacetic acid was mixed with 980 µL diluent (dilution 1/50) (Final concentration: about 50 µg/mL guanidinoacetic acid). |
| Spiked samples*: | 20 µL SØ guanidinoacetic acid was mixed with 100 µL S2 calibration stock solution and 880 µL diluent. |
| | (Final concentration: about 50 µg/mL guanidinoacetic acid and 50ng/mL dicyanodiamide and/or 50 ng/mL melamine). |

| | |
|---|---|
| *When the content of dicyandiamide (or melamine) was expected above the highest calibration point (when using the calibration series shown in table 1 above this is about 300 ng/mL), the sample solutions had to be further diluted. | |

When the content of melamine (or dicyandiamide) was expected below the lowest calibration point (when using the calibration series shown above this is about 10 ng/mL) the pipetting scheme for the calibrants scheme had to be adopted to the expected content range down to about 5 ng/mL (lowest possible calibration point: 20 µL S3 + 980 µL diluent).

The finally applied dilution and the used calibration were documented.

### 3.2 Description of the HPLC method

| | |
|---|---|
| Column: | 50 x 2.1 mm Acclaim Trinity P2 (Thermo Fisher), the end of the HPLC column was hooked up to the mass spectrometer. |
| Eluent A: | 20 mM ammonium formiate in water |
| Eluent B: | acetonitrile |

### Gradient table:

| **Time (min)** | **% A** | **% B** | **Flow mL/min** |
|---|---|---|---|
| 0 | 5 | 95 | 0.3 |
| 2 | 5 | 95 | 0.3 |
| 12 | 80 | 20 | 0.3 |
| 17 | 80 | 20 | 0.3 |

| | |
|---|---|
| Autosampler rack: | room temperature |
| Column temperature: | 40 °C |
| Injection volume: | 10 µL |

### 3.3 Setup and tuning of the mass spectrometer system

MS tuning was started with a purified transfer capillary and tuning solution was applied. The values in table 2 below (especially the values related to the ion source parameters and the collision energy) were considered as initial value for tuning. Any change of the MS hardware (even when equivalent) had to be considered as requiring adaptations of tuning parameters.

| | |
|---|---|
| Instrument: | QQQ 6430 Mass spectrometer system (Agilent Technologies) |
| Detection mode: | Electrospray ionization in cation mode |
| Capillary voltage: | 3000 volts |
| Gas temperature: | 350 °C |
| Gas flow: | 13 L/min |
| Nebulizer: | 50 psi |
| Scan function: | MRM |

**Table 2: Ion transitions for MRM**

| **Substance** | **Fragmentor** | **Precusor ion [m/z]** | **Product ion [m/z]** | **Collision energy** | **Cell Accelerator Voltage** | **Retention time [min]** |
|---|---|---|---|---|---|---|
| Dicyandiamide | 43 | 85.1 | 68.0 | 20 | 1 | 1.5 |
| Melamine | 118 | 127.1 | 85.1 | 20 | 1 | 5.5 |

### 3.4 Operational procedure

The mass spectrometer batch table was edited according to the user's manual of the manufacturer. All prepared blanks, calibrants, samples and spiked samples were processed block-wise under full autosampler control. The samples were arranged in the autosampler tray in the following sequence order:
The system was equilibrated with three blank injections (diluent only). Then the sample sequence was started.

For each sample batch, two independently prepared replicates of un-spiked sample solutions were injected. Within the sample queue at least one spiked sample solution was prepared for every 10^{th} sample as quality control sample.

Calibration series were injected at the beginning and at the end of the sample queue (bracketing). For long sample queues additional calibrations were included in between.

The complete sample series had to be measured without ramping down the instrument within the series.

### 3.5 System suitability testing

The system suitability for the applicability of the chromatographic system as well as for the mass spectrometric detection could be taken directly from the measurement of the calibration series as well as of at least one spiked sample measured in each sample queue. Table 3 summarizes the acceptance criteria for the system suitability.

**Table 3: The acceptance criteria for system suitability**

| **Parameter** | **Criteria** |
|---|---|
| Retention time of dicyandiamide | 1.5 min ± 0.2 min |
| Retention time of melamine | 5.5 min ± 0.5 min |
| Quadratic correlation coefficient of quadratic regression of the calibration curves | R² > 0.995 (calculated for at least 5 values; the targeted concentration range had to be included) |
| Recovery for reference compounds in spiked QC samples | Recovery: 80 - 120% (spiked amount must be within the targeted range of calibration, e.g., calibration step 3 or 4) |

### 3.6 Evaluation

The quantification of dicyandiamide and melamine was based in external calibration averaged from calibrant series before and after the sample injection (bracketing calibration). The calibration is calculated by a quadratic calibration curve (weighing factor 1/x).

### II. Quantitative Determination of dicyandiamide in guanidinoacetic acid

Dicyandiamide was quantitatively determined both in chemically produced guanidinoacetic acid (from Gendone, China, identified as Chem GAA) and in the guanidinoacetic acid according to the present invention (from cultivation of a suitable microorganism, identified as Bio GAA) using the method described under point I above.

The following samples were prepared and subjected to two reparative experiments according to the procedure under point I above.

**Table 4: Overview over the samples**

| **Sample ID** | **Sample name** | **Lot** |
|---|---|---|
| S-220818-00936 | Chem GAA, AU 220214/3596 | 220519HG31 |
| S-220818-00937 | Chem GAA, AU 220244/3597 | 220617HG31 |
| S-220818-00938 | Chem GAA, AU 220245/3598 | 220618GUB1 |
| S-220818-00939 | Chem GAA, AU 220387/3599 | 220715HG31 |
| S-220818-00940 | Bio GAA, AU 220251/3600 | AR2200052 |

The results for the quantitative determination of dicyandiamide are summarized in table 5 below:

**Table 5: Results for the quantitative determination of dicyandiamide**

| **Sample** | **Sample ID** | **Dicyandiamide** | |
|---|---|---|---|
| | | **ppm [mg/kg]** | **[%]** |
| Chem GAA, AU 220214/3596 | S-220818-00936 | 647 | 0.06 |
| | | 581 | 0.06 |
| Chem GAA, AU 220244/3597 | S-220818-00937 | 835 | 0.08 |
| | | 694 | 0.07 |
| Chem GAA, AU 220245/3598 | S-220818-00938 | 638 | 0.06 |
| | | 580 | 0.06 |
| Chem GAA, AU 220387/3599 | S-220818-00939 | 689 | 0.07 |
| | | 582 | 0.06 |
| Bio GAA, AU 220251/3600 | S-220818-00940 | < cal (< 8 ppm) | < cal (< 0.0008 ppm) |
| | | < cal (< 8 ppm) | < cal (< 0.0008 ppm) |

No dicyandiamide could be detected in the biochemically produced guanidinoacetic acid.

### III. Quantitative Determination of melamine, cyanuric acid, ammelin and ammelid in guanidinoacetic acid

Melamine, cyanuric acid, ammelin, and ammelid were quantitatively determined both in chemically produced guanidinoacetic acid (from Gendone, China, identified as Chem GAA) and in the guanidinoacetic acid according to the present invention (from cultivation of a suitable microorganism, identified as Bio GAA) using the method described in Laboratory Information Bulletin (LIB) 4422: Melamine and Cyanuric Acid Residues In Foods (FDA LIB No. 4422, October 2008).

Tables 6 and 7 below summarize the results for determination in chemically produced guanidinoacetic acid (Chem GAA) and biochemically produced guanidinoacetic acid (Bio GAA).

**Table 6: Results for melamine, cyanuric acid, ammelin, and ammelid in Chem GAA**

| **Parameter** | **Results [mg/kg]** |
|---|---|
| melamine | > 2 |
| cyanuric acid | < 0.1 |
| ammelin | 0.24 |
| ammelid | 0.13 |

**Table 7: Results for melamine, cyanuric acid, ammelin, and ammelid in Bio GAA**

| **Parameter** | **Results [mg/kg]** |
|---|---|
| melamine | n. d. (< 0.1) |
| cyanuric acid | n. d. (< 0.1) |
| ammelin | n. d. (< 0.1) |
| ammelid | n. d. (< 0.1) |

Melamine, cyanuric acid, ammelin, and ammelid could be detected in the chemically produced GAA. However, no melamine, cyanuric acid, ammelin or ammelid could be detected in the Bio GAA. The limit of detection of melamine, cyanuric acid, ammelin and ammelid was 0.1 ppm.

## Claims

1. Guanidinoacetic acid, **characterized in that** it comprises less than 0.3 ppm of melamine and/or less than 600 ppm of dicyandiamide and/or cyanamide as impurity or secondary component, determined by LC-MS/MS.

2. Guanidinoacetic acid according to claim 1, wherein the guanidinoacetic acid comprises not more than 0.1 ppm of melamine, determined by LC-MS/MS.

3. Guanidinoacetic acod according to claim 1 or 2, wherein the guanidinoacetic acid comprises less than 0.1 ppm of ammeline, determined by LC-MS/MS.

4. Guanidinoacetic acid according to any of claims 1 to 3, wherein the guanidinoacetic acid comprises less than 0.1 ppm of ammelide, determined by LC-MS/MS.

5. Guanidinoacetic acid according to any of claims 1 to 4, wherein the guanidinoacetic acid comprises less than 0.1 ppm of cyanuric acid, determined by LC-MS/MS.

6. Guanidinoacetic acid according to any of claims 1 to 5, wherein the guanidinoacetic acid comprises less than 500 ppm of dicyandiamide and/or cyanamide, determined by LC-MS/MS.

7. Guanidinoacetic acid according to any of claims 1 to 6, wherein the guanidinoacetic acid is obtained by a process according to any of claims 8.

8. Process for producing guanidinoacetic acid according to any of claims 1 to 7, comprising the step of cultivating a microorganism comprising at least one gene coding for a protein having the function of an L-arginine:glycine amidinotransferase in the presence of one or more carbon sources, wherein at least one carbon source comprises biogenic carbon.

9. Process according to claim 8, wherein the at least one carbon source comprising biogenic carbon comprises or consists of a sugar.

10. Process according to claim 8 or 9, wherein the microorganism further comprises at least one gene coding for a protein having the function of an NADH-dependent amino acid dehydrogenase.

11. Process according to claim 8 or 9, wherein the microorganism further comprises at least one protein having the function of a glyoxylate aminotransferase.

12. Process according to claim 8 or 9, wherein the activity of a protein having the function of a malate synthase is decreased compared to the respective activity in the wildtype microorganism.

13. Process according to claim 8 or 9, wherein the expression of a gene encoding the protein having the function of a malate synthase is attenuated compared with the expression of the respective gene in the wildtype microorganism or a gene encoding the protein having the function of a malate synthase is deleted.

14. Process according to claim 8 or 9, wherein the microorganism further comprises an increased activity of an enzyme having the function of a glyoxylate aminotransferase compared to the respective enzymic activity in the wildtype microorganism.

15. Feed additive comprising or consisting of the guanidinoacetic acid according to any of claims 1 to 7 or the guanidinoacetic acid obtained by the process according to any of claims 8 to 14.
